(19) Europäisches Patentamt
European Patent Office
Office européen des brevets

(11) EP 2 384 697 A1

(12) **EUROPEAN PATENT APPLICATION**

(43) Date of publication:
**09.11.2011 Bulletin 2011/45**

(51) Int Cl.:
*A61B 5/08* (2006.01)        *A61B 5/085* (2006.01)
*G06F 19/00* (2011.01)

(21) Application number: **10161953.4**

(22) Date of filing: **05.05.2010**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(71) Applicants:
• **Universiteit Gent**
  **9000 Gent (BE)**
• **VRIJE UNIVERSITEIT BRUSSEL**
  **1050 Brussel (BE)**

(72) Inventors:
• **Ionescu, Clara**
  **9052, Zwijnaarde (BE)**
• **De Keyser, Robain**
  **9880, Aalter (BE)**
• **Schoukens, Johan**
  **1050, Brussel (BE)**

(74) Representative: **Hertoghe, Kris Angèle Louisa et al**
  **DenK iP bvba**
  **Hundelgemsesteenweg 1114**
  **9820 Merelbeke (BE)**

(54) **Method and device for determining non-linear effects in the respiratory impedance**

(57)     The present invention is directed to a method and device for measuring the respiratory impedance over low frequencies, e.g. in a range between 0.1 and 50 Hz. The present invention provides a method for determining non-linear effects in respiratory impedance of a subject having a respiratory system. The method comprises constructing an excitation signal comprising a plurality of excitation frequencies, applying the constructed excitation signal to an actuator, e.g. an FOT device, for applying pressure oscillations to the respiratory system of the subject, measuring the frequency response function of the respiratory impedance and performing a frequency analysis, and determining presence of non-excited harmonics in the frequency response function.

FIG. 1

EP 2 384 697 A1

## Description

### Field of the invention

The present invention is directed to a method and device for measuring respiratory impedance. In particular, the present invention relates to a method and device for determining non-linear effects in the respiratory impedance. More particularly, the present invention is directed to a method and device for measuring the respiratory impedance over low frequencies, e.g. in a range between 0.1 and 50 Hz, for example between 0.1 and 25 Hz, e.g. between 0.1 and 22 Hz, which can provide an indication of pathology of respiratory tissues.

### Background of the invention

The frequency response function of the respiratory impedance has been extensively studied over the last decades. New technologies for data processing and new methods for analysis, diagnosis and simulation of the human respiratory system have contributed to the progress in this field. An increase in interest has occurred for the low frequency behavior of the respiratory impedance.

The forced oscillation technique (FOT) is an old non-invasive technique for determining the impedance of the respiratory system by applying sinusoidal pressure variations to the respiratory system. The respiratory impedance can then be determined by the ratio of the measured air pressure and air flow at the mouth of the patient. The respiratory impedance provides information about the elastic, inertial and resistive properties of the respiratory system. Over time, the technique has evolved in terms of measurement devices, excited oscillation frequencies and evaluation principles.

During these prior art measurements, the respiratory system and the related measurement instrumentation are assumed to be linear, because of the simplicity of the linear framework. However, in practice the respiratory system and the measurement devices are not linear, and often one is not aware of the presence of non-linear distortions. The question arises to what extent these non-linearities affect the obtained results and whether these are still reliable.

### Summary of the invention

It is an object of embodiments of the present invention to provide a good method and device for determining the respiratory impedance of the respiratory system.

The above objective is accomplished by a method and device according to the present invention.

In a first aspect, the present invention provides a method for determining non-linear effects in respiratory impedance of a subject having a respiratory system. The method comprises constructing an excitation signal comprising a plurality of excitation frequencies, applying the constructed excitation signal to an actuator, e.g. an FOT device, for applying pressure oscillations to the respiratory system of the subject, measuring the frequency response function of the respiratory impedance and performing a frequency analysis, and determining presence of non-excited harmonics in the frequency response function.

It is an advantage of embodiments of the present invention that non-linear effects of the respiratory system are taken into account. In the past, people have always tried to avoid non-linearities; however, as the biological system, and in particular the respiratory system, is non-linear by definition, and the non-linearity changes with pathology, the measured non-linearities may be useful or interesting from a clinical standpoint.

It is an advantage of embodiments of the present invention that the results can be used for analysis of viscoelastic properties of the respiratory tissues. It is an advantage of embodiments of the present invention that the non-linear disturbances of the respiratory analysis system allow to determine a respiratory system pathology.

A method according to embodiments of the present invention may furthermore comprise determining a breathing frequency of the subject. In embodiments of the present invention, determining a breathing frequency of the subject may comprise, before any measurement with an excitation signal, measuring the frequency response function of the respiratory impedance with no externally applied input signal. This provides a measurement of the breathing of the subject, usually at low frequencies, around 0.15-0.25Hz. The energy of this signal in its spectral power density function will give information upon the breathing frequency. Measuring the frequency response function of the respiratory impedance with no externally applied input signal may be performed during a few breathing periods. The measurement may for example be performed during a few seconds, e.g. 3 to 4 breathing periods, fairly similar to a wavelength of a sinusoidal signal.

Constructing an excitation signal in a method according to embodiments of the present invention may comprise constructing a signal comprising frequencies different from the breathing frequency and harmonics thereof. This way, correlation between breathing and excitation signals is avoided. A patient-individualized excitation signal is constructed this way, by first measuring a patient dependent breathing signal and then using frequencies different from the breathing frequency and harmonics thereof when constructing the excitation signal.

In a method according to embodiments of the present invention, constructing an excitation signal may comprise constructing a multisine. A multisine is a periodic signal consisting of the sum of a plurality of sine waves with user-defined amplitudes. By using a multisine, the input frequencies can be selected very precisely.

In accordance with embodiments of the present invention, the multisine may be a random phase odd multisine with random harmonic grid defined by

$$u_{odd,r}(t) = \sum_{k=1}^{\frac{N}{2}-1} A_{2k-1} \cos\left((2k-1)\omega_0 t + \Phi_{2k-1}\right)$$

In a method according to embodiments of the present invention, constructing an excitation signal may comprise

- selecting a frequency resolution $f_0$, excited harmonics k, amplitudes $A_k$, and the root mean square value of the excitation signal, and
- making a random choice of the phases of the non-zero harmonics of the excitation signal such that the variance of the phase is uniformly distributed in the $0$-$2\pi$ interval and obtaining the excitation signal.

Constructing an excitation signal may comprise constructing a signal in a frequency range between 0.1 Hz and 50.0 Hz, such as between 0.1 Hz and 25.0 Hz, e.g. between 0.1 and 22 Hz. This frequency range is particularly advantageous, as the non-linear effects in this frequency range can be measured. At higher frequencies (> 50 Hz) the non-linear effects are very small and not very interesting. It is particularly useful for clinical insight (viscoelastic phenomena) to stay in the range up to 25 Hz.

A method according to embodiments of the present invention may further comprise, at least once, and preferably a plurality of times, repeating the steps of constructing an excitation signal, applying the constructed excitation signal, measuring the frequency response function of the respiratory impedance and determining presence of non-excited harmonics in the frequency response function. In a particular embodiment, the constructed excitation signals may be different in phase but not in amplitude. By repetition of the same process with excitation signals with different parameters, non-linearities can be better and better determined, hence better and more reliable results are finally obtained

Constructing an excitation signal may comprise optimizing the excitation signal by reducing non-linear effects of measurement instrumentation. This may be obtained for example by avoiding excitation frequencies in the constructed excitation signal which lead to non-linearities in the measured signal due to non-linear behavior of the measurement instrumentation. Such non-linear behavior of the measurement instrumentation and excitation frequencies responsible for this non-linear behavior can be determined before starting an actual measurement on a subject.

A method according to embodiments of the present invention may comprise applying signal processing methods, such as for example filtering and/or a specific design of the input signal to be uncorrelated with breathing, to minimize the effect of noise introduced by the breathing of the subject on estimated variables. For example, for avoiding exciting with input signals at harmonics which are related to breathing frequency, first a measurement may be performed without external excitation signal (only breathing), after which the frequency of the breathing may be estimated, and then an excitation signal may be constructed not containing this breathing frequency nor harmonics thereof. Alternatively, a randomly generated excitation signal may be filtered so as to filter out the breathing frequency and harmonics thereof, before applying this signal to the FOT device. This way, a patient-specific excitation signal is obtained, hence patient specific results can be obtained.

A method according to embodiments of the present invention may furthermore comprise evaluating the results for the respiratory impedance. This respiratory impedance can be an input parameter for a medical doctor.

In a method according to embodiments of the present invention, constructing an excitation signal may comprise determining an excitation interval, e.g. an excitation interval in the range between 0.1 and 50 Hz, for example between 0.1 and 25 Hz, e.g. between 0.1 and 22 Hz,

determining a tuning parameter in the excitation interval, e.g. steps of 0.1 Hz,

selecting a number of measurement points (excitation frequencies) within the excitation interval, e.g. between 3 and 7, for example 3 or 4, corresponding to the tuning parameter but excluding the breathing frequency and harmonics thereof. The excitation frequencies may be selected randomly over the excitation interval.

Constructing an excitation signal may comprise splitting the excitation interval in sub-intervals of a pre-determined number of excitation points, e.g. 3 or 4 excitation points, and leaving one excitation point out randomly from each sub-interval.

In a method according to embodiments of the present invention, measuring the frequency response function of the respiratory impedance may comprise measuring the frequency response function from a plurality of consecutive periods of a steady state response. As the excitation signal comprises a plurality of excitation frequencies, in particular may be a multisine, it may be advisable to select a long enough measurement time in order to obtain good measurement results.

A method according to embodiments of the present invention may comprise repeating the steps of constructing an excitation signal, applying the constructed excitation signal to an actuator, and measuring the frequency response function of the respiratory impedance and performing a frequency analysis.

In a second aspect, the present invention provides a controller for providing an excitation signal to an FOT device, the controller being programmed for providing an excitation signal having frequency components in the frequency range 0.1 to 50 Hz, for example 0.1- 25 Hz, e.g. between 0.1 and 22 Hz. The controller may be adapted for performing any of the method steps as cited above.

In embodiments of the present invention, a controller is provided for determining non-linear effects in respiratory impedance of a subject having a respiratory system, the controller being programmed for constructing an excitation signal comprising a plurality of excitation frequencies, for applying the constructed excitation signal to an actuator for applying pressure oscillations to the respiratory system of the subject, for measuring the frequency response function of the respiratory impedance and performing a frequency analysis, and for determining presence of non-excited harmonics in the frequency response function.

In a third aspect, the present invention provides an FOT device comprising a controller in accordance with embodiments of the second aspect of the present inveiton. The controller may be programmed so as to excite an actuator with an excitation signal having frequency components in the frequency range 0.1 to 50 Hz, for example 0.1- 25 Hz, e.g. between 0.1 and 22 Hz.

In embodiments of the third aspect, the FOT device may be loudspeaker based.

In alternative embodiments of the third aspect, the FOT device may be pump-based. An FOT device according to embodiments of the present invention may comprise a piston based pump for generating a pressure oscillation for being applied to a subject. Such pump-based FOT device is particularly useful for the 0.1 to 25 Hz excitation frequency range.

A pump-based FOT device may be advantageous over a loudspeaker FOT device in that it may be made with smaller dimensions.

In a fourth aspect, the present invention provides a computer program product for executing any of the methods according to the first aspect when executed on a controller associated with an FOT device.

The present invention furthermore provides a machine readable data storage storing the computer program product of claim 40.

Particular and preferred aspects of the invention are set out in the accompanying independent and dependent claims. Features from the dependent claims may be combined with features of the independent claims and with features of other dependent claims as appropriate and not merely as explicitly set out in the claims.

For purposes of summarizing the invention and the advantages achieved over the prior art, certain objects and advantages of the invention have been described herein above. Of course, it is to be understood that not necessarily all such objects or advantages may be achieved in accordance with any particular embodiment of the invention. Thus, for example, those skilled in the art will recognize that the invention may be embodied or carried out in a manner that achieves or optimizes one advantage or group of advantages as taught herein without necessarily achieving other objects or advantages as may be taught or suggested herein.

**Brief description of the drawings**

FIG. 1 illustrates an FOT-device according to a first embodiment of the present invention as may be used for implementing embodiments of the present invention, wherein u(t) is an excitation signal for applying a forced oscillation to the respiratory system, q(t) is the air flow at the mouth of a subject, and p(t) is the air pressure at the mouth of the subject. FIG. 1 illustrates a loudspeaker-driven setup.

FIG. 2 illustrates the respiratory impedance for a healthy subject obtained with FOT, both on a prior art device and on a device in accordance with embodiments of the present invention.

FIG. 3 illustrates an odd multisine which may be used as an excitation signal in accordance with embodiments of the present invention in time domain (left hand side) and in frequency domain (right hand side).

FIG. 4 illustrates the principle of detecting non-linearities.

FIG. 5 illustrates input u(t) and output y(t) measurements with noise of a non-linear system actuated by an actuator with input signal r(t) (top part), and a corresponding BLA (bottom part).

FIG. 6 schematically illustrates a measurement procedure for a robust method according to embodiments of the present invention.

FIG. 7 schematically illustrates a measurement procedure for a fast method according to embodiments of the present invention.

FIG. 8a illustrates measurement of the best linear approximation (BLA) of the device impedance $Z_{device}$ of a prior art device (left) and of a device according to embodiments of the present inventioin (right) on a calibration tube.

FIG. 8b illustrates measurement of the BLA of the impedance $Z_{device}$ of a loudspeaker-driven device (left) and of a piston-driven device (right), both according to embodiments of the present invention.

FIG. 9 illustrates measurement of the best linear approximation (BLA) of the device impedance $Z_{device}$ of a prior art device with odd multisine $U_{odd}$ (top) and even multisine $U_{even}$ (bottom).

FIG. 10 illustrates measurement of the best linear approximation (BLA) of the device impedance $Z_{device}$ of a prior art device with ½ $U_{odd}$ (top) and $2U_{odd}$ (bottom).

FIG. 11 illustrates input (left column), output (middle column) and corrected DFT spectrum (right column) of a healthy patient of 9 years old.

FIG. 12 illustrates input (left column), output (middle column) and corrected DFT spectrum (right column) of a healthy patient of 7 years old.

FIG. 13a and FIG. 13b illustrate input (left column), output (middle column) and corrected DFT spectrum of a non-smoker and of a smoker, respectively.

FIG. 14 illustrates measurement of the best linear approximation (BLA) of the respiratory impedance $Z_{respiratory}$ of a smoker (top) and non-smoker (bottom).

FIG. 15 shows a frequency characteristic of a loudspeaker.

FIG. 16 shows a schematic overview of a system in accordance with embodiments of the present invention for the generation of a signal to a stepper motor.

FIG. 17 illustrates an FOT-device according to a second embodiment of the present invention as may be used for implementing embodiments of the present invention, wherein u(t) is an excitation signal for applying a forced oscillation to the respiratory system, q(t) is the air flow at the mouth of a subject, and p(t) is the air pressure at the mouth of the subject. FIG. 17 illustrates a piston-driven setup.

FIG. 18 illustrates the BLA of a calibration pipe, for one realization, hence fast method (left) and for 4 measurements, hence robust method (right).

The drawings are only schematic and are non-limiting. In the drawings, the size of some of the elements may be exaggerated and not drawn on scale for illustrative purposes.

Any reference signs in the claims shall not be construed as limiting the scope.

In the different drawings, the same reference signs refer to the same or analogous elements.

**Detailed description of illustrative embodiments**

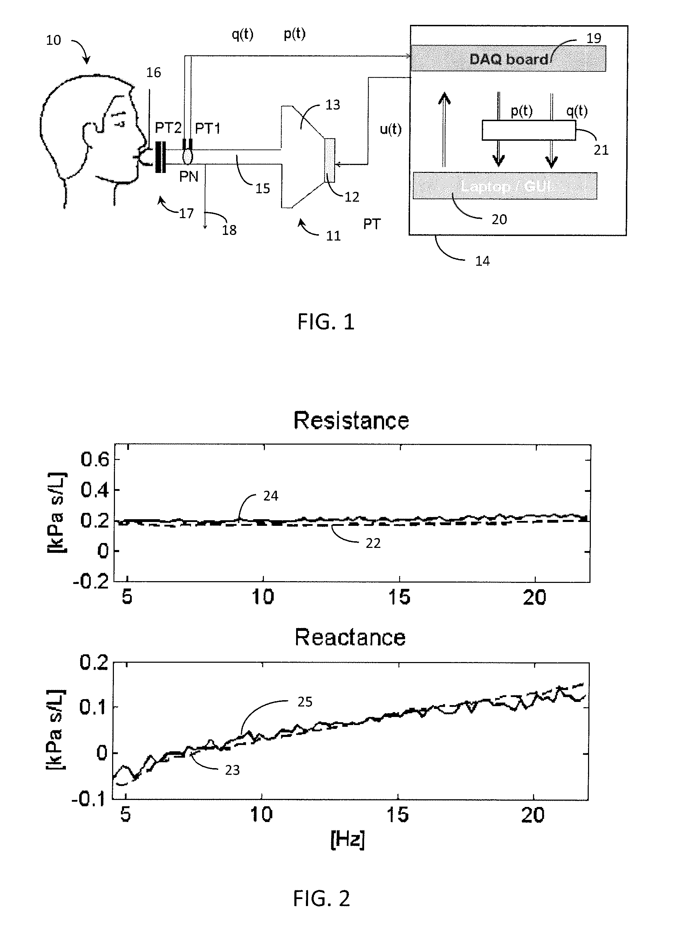

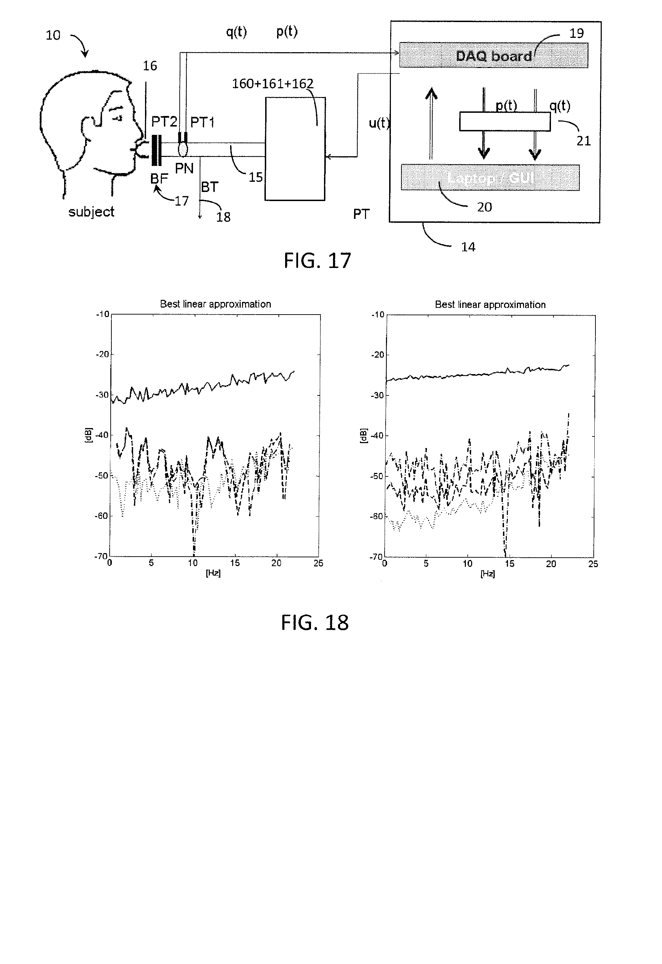

A schematic illustration of a forced oscillation technique (FOT) device for performing the forced oscillation technique, as may be used in embodiments of the present invention, is given in FIG. 1 and FIG. 17. The FOT device applies small pressure oscillations to the respiratory system of a subject 10 under test, breathing spontaneously. It is an advantage of FOT that it does not require forced breathing from the subject 10 under test.

In the implementation according to FIG.1, pressure oscillations are generated by a loudspeaker 11 comprising a loudspeaker cone 12 connected to a chamber 13. The loudspeaker cone 12 is driven by a power amplifier (not illustrated in detail) fed with an oscillating excitation signal u(t) generated by a controller 14. The movement of the loudspeaker cone 12 generates a pressure oscillation inside the chamber 13, which is applied to the subject's respiratory system by means of a tube 15 connecting the loudspeaker chamber 13 to a mouthpiece 16 for connecting the tube 15 via the mouth to the subject's respiratory system. For safety of the subject 10, the pressure of the excitation signal u(t) should be kept within a range of a peak-to-peak size of 0.1- 0.3 kPa. Optionally, an anti-bacterial filter 17 may be provided between the tube 15 and the mouthpiece 16, for avoiding infections to be transferred from one subject to another during subsequent measurements with a same FOT device. A bias tube 18 allows the subject 10 to breath fresh air during the measurements. The bias tube 18 is a branch of the tube 15 with on the one hand a sufficiently low resistance at low frequencies of the excitation signal u(t) , e.g. between 0.1 and 50 Hz, for example between 0.1 and 25 Hz, such as between 0.1 and 22 Hz, to allow for a spontaneous breathing by the subject 10, but on the other hand a sufficiently high resistance to avoid pressure oscillations from the loudspeaker 11 to be removed via the bias tube before they ever reach the respiratory system of the subject 10.

Air pressure p(t) and air flow q(t) may be measured at the mouthpiece 16 by any suitable sensor or sensor unit, for example by a combination of two pressure sensors PT1, PT2 and a pneumotachograph PN. As an example only, by using a pneumotachograph PN, comprising two pressure sensors PT1, PT2 over a known resistance, the air flow q(t) at the subject's mouth can be determined. In this configuration, the pressure sensor PT2 closest to the subject's mouth also measures the corresponding air pressure p(t) at the subject's mouth.

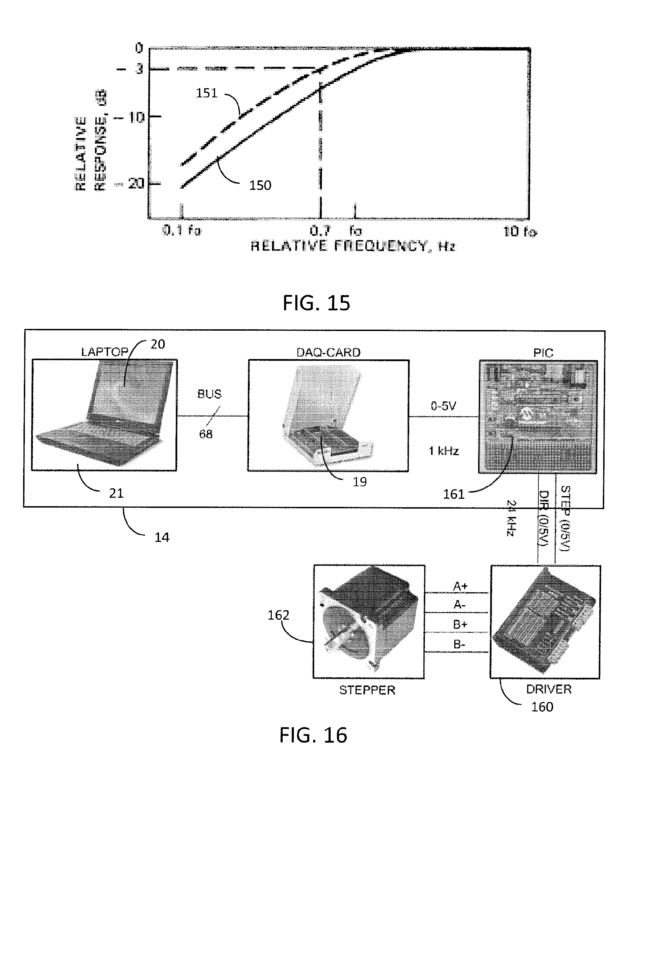

Although a loudspeaker set-up as illustrated above would work, it has some disadvantages. FIG.15 depicts a spectral power density plot for a loudspeaker, whereby graph 150 depicts theoretical results, and graph 151 represents measurements. It can be seen that the power to generate a sufficiently-strong excitation signal at low frequencies, i.e. frequencies around or below the resonant frequency of the loudspeaker, diminishes significantly. In principle, a bigger size loudspeaker would be able to produce the required power at low frequency oscillations, e.g. oscillations in the range of 0.1 to 50 Hz, for example in the range of 0.1 to 25 Hz, e.g. between 0.1 and 22 Hz; however, since it is advantageous to keep the device at its minimal size/weight/cost, a different solution to produce air pressure oscillations has also been considered.

A schematic of another device according to embodiments of the present invention is depicted in FIG. 16, where it is possible to identify the following elements: a controller 14 comprising a computing element 21, optionally with graphical interface 20, for sending a desired excitation signal, e.g. a multisine signal, as an analogue output (in Volts) from a data acquisition board 19 (e.g. from National Instruments, 6036E series, 12 bit, 200ksamples/second) as a driving signal to a motor driver 160, e.g. a stepper motor driver. The voltage may be transformed via a programmable PIC element 161 into pulses which are applied to the motor driver 160, e.g. stepper motor driver, and which may have the meaning of 'number of steps' which go to a motor 162, e.g. a stepper motor. The pulses may be characterized by a sign (direction of motor rotation) and number of steps (how many 'teeth' the stepper motor 162 has to move). A piston based pump (not illustrated) may be connected to the stepper-motor 162 may be connected to the FOT device as illustrated in FIG. 1 as a replacement of the loudspeaker illustrated there, in order to provide low frequency measurements; the frequency range may then for example be 0.1 to 50 Hz, for example 0.1-25 Hz, e.g. between 0.1 and 22 Hz. The movement of the motor 162 causes a movement in the air reservoir (pneumatic piston), which in turn causes air pressure oscillations. In particular embodiments, all parts are in plastic, hence isolation is provided for patient and/or user safety.

In a particular embodiment, the dimensions of the box comprising the air reservoir are 0.3 x 0.3 x 0.3 m. The resolution of the flow which can be produced with such a system is 0.021 l/s, whereas the resolution of the pneumotachograph used (in this particular case Hans Rudolph, series 4830B, nr 112917) is 0.045 l/s; thus, very accurate. The device can send a pure sinusoid at peak to peak amplitude of 0.35 kPa starting from 0.625Hz. In case of a multisine excitation signal, since the peak-to-peak amplitude of the sum of sinusoids has to be within the 0.35kPa, the device has no difficulty to produce the required amplitude.

An representation of a piston-driven FOT device is schematically illustrated in FIG. 17. In this embodiment, a piston is moving in order to create air movement as a result of the motor movement.

The sensor units, e.g. the pressure/flow sensors may be provided as explained with reference to FIG. 1 for measuring air pressure p(t) and air flow q(t) at the mouth of the subject 10. The pressure sensors may for example by sensors from SensorTechnics, HCXPM005D6 (BSDX series) or similar.

In both cases illustrated (FIG. 1 and FIG. 17), the measured signals p(t), q(t) are sent to the controller 14, for example to a data acquisition board 19 thereof, for signal processing. The measured signals p(t), q(t) may be sent to the controller 14 via a low-pass filter (not illustrated). The controller 14 controls the operation of the FOT device and controls the signal processing. The controller 14 may include a computing device 21, e.g. a microprocessor, for instance it may be a microcontroller. In particular, it may include a programmable FOT device controller, for instance a programmable digital logic device such as a Programmable Array Logic (PAL), a Programmable Logic Array, a Programmable Gate Array, especially a Field Programmable Gate Array (FPGA). The use of an FPGA allows subsequent programming of the FOT device, e.g. by downloading the required settings of the FPGA. The controller 14 illustrated in FIG. 1 and FIG. 16 includes a data acquisition board 19 for receiving measurement values of air flow q(t) and air pressure p(t) and a graphical user interface 20 for interfacing with an application running on the controller 21.

From the measured air pressure p(t) and air flow q(t) at the mouth of the subject 10 during normal or spontaneous breathing, the respiratory impedance $Z_r(j\omega)$ can be determined. The respiratory impedance $Z_r(j\omega)$ provides the ratio between the air pressure p(t) and the air flow q(t) measured at the mouth of the subject 10 during normal or spontaneous breathing, at different excitation frequencies of the excitation signal u(t). The respiratory impedance $Z_r(j\omega)$ characterizes the balance between elastic, inertial and resistive characteristics of the respiratory system. In case of a lung pathology this balance is changed, depending on the specific pathology and the corresponding typical changes in lung tissue, hence also the respiratory impedance $Z_r(j\omega)$ is changed. It has been found by the present inventors that if the excitation signal u(t) used for identification of the respiratory impedance $Z_r(j\omega)$ is suitably selected, i.e. is selected in the low frequency range, e.g. between 0.1 and 50 Hz, for example between 0.1 and 25 Hz, such as between 0.1 and 22 Hz, the effect of the changes in the balance between mechanical parameters of the respiratory system can be quantified.

In experiments to prove the value of the present invention, measurements have been made on three FOT-devices:

- A first FOT device is the IRs8: This is a loudspeaker-based prototype device according to embodiments of the present invention, designed for being excited in the frequency range 0.5 - 21.9 Hz.
- A second FOT device is the I2M: This is a prior art FOT device designed for being excited in the frequency range 4 - 48 Hz.

- A third FOT device is a piston-based prototype device according to embodiments of the present invention.

The loudspeaker-based and particularly the piston-based prototype devices according to embodiments of the present invention were designed for applying excitation signals u(t) with a lower frequency range than the frequency range which is traditionally applied in prior art devices. These lower frequencies hold valuable information from medical point of view, since they are closer to the breathing signal and thus give valuable information about the respiratory mechanics in the frequency range of the breathing (typically between 0.1 and 0.3 Hz).

In FIG. 2 the respiratory resistance and reactance of a subject 10 are obtained with a piston-driven device according to an embodiment of the present invention (graphs 24 and 25, respectively) and with a prior art I2M device (graphs 22 and 23, respectively). The respiratory impedance is defined as the ratio of the measured air pressure and air flow at the mouth of the patient. In FIG. 2, the results look nearly the same, although the results with the piston-driven device according to an embodiment of the present invention (graphs 25, 25) appear to be slightly noisier. It has to be noted that this FIG. 2 only illustrates results in the frequency range 4 to 22 Hz, as both devices can function in this frequency range without disturbances by low pass or high pass filters. This frequency range is only used for comparison of both devices. In experiments, the prototype devices according to embodiments of the present invention will be used to assess respiratory impedance also at frequencies below 4Hz, in accordance with embodiments of the present invention.

The fundamental difference between a linear time invariant system and a nonlinear time invariant system is that the nonlinear system transfers energy from one frequency in the excitation signal to other frequencies in the output signal.

The influence of nonlinear distortions on frequency response function (FRF) measurements may be determined by using a random phase multisine as excitation signal, comprising a predetermined number N of sinusoidal signals, N being a design parameter:

$$\text{u(t)} \quad = \sum_{k=1}^{N} A_k cos(k\omega_0 t + \phi_k)$$

In accordance with embodiments of the present invention, the excitation signal u(t) consists of a random phase multisine with random harmonic grid. Random phase multisines are periodic signals consisting of the sum of N harmonically related sine waves with user-defined amplitudes $A_k$ and random phases $\Phi_k$. A specially designed excitation signal u(t) according to embodiments of the present invention is a random phase odd multisine given by:

$$u(t) = \sum_{k=1}^{N/2-1} A_{2k-1}(\cos(2k-1)2\pi f_0 t + \phi_{2k-1}$$

where in-band harmonics (=some of the detection lines) are randomly omitted.

To guarantee a minimal resolution of the detection lines, in accordance with embodiments of the present invention, the excited harmonics are split into groups of equal number of consecutive lines, and one harmonic randomly chosen from the group is eliminated. For example, a hundred excited harmonics (e.g. between 0.1 Hz and 10.1 Hz, excitations every 0.1 Hz) may be split into 25 groups of four consecutive excited harmonics, and one out of four harmonics is randomly omitted. In this case the design parameter N is a random number between 25 and 51, based on the minimum and maximum number of excited lines in the interval.

The amplitudes $A_k$ of the harmonics, the frequency distribution $f_k$ of the harmonics (uniform or logarithmic, in function of how many decades are investigated), the type of the excited harmonics (odd or even and odd) and the number of consecutive harmonics in each group $F_{group}$, may be set by the user, for example by suitably programming the controller 14. Hence, these are the design parameters. The design parameters may be determined as follows:

- the amplitudes may be determined such that the sum of all sines does not exceeed the maximum allowed for patient safety,
- the frequency distribution $f_k$ of the harmonics may be infinite,
- the number of consecutive harmonics $F_{group}$ in each group may be optimal for 3 or 4.

As to the type of excited harmonics, an odd multisine is a multisine in which only odd harmonics of $f_0$ are excited (k = odd). An even multisine is a multisine in which only even harmonics of $f_0$ are excited (k = even). In a full multisine, both even and odd harmonics of $f_0$ are excited.

In case of an even multisine as excitation signal, the corresponding non-linearities do not contribute to the best linear approximation of the non-linear system considered because with an even number of frequency combinations it is possible to cancel the random phases. Hence applying an even multisine is a less optimal choice for embodiments of the present invention. An advantage of applying an odd multisine as excitation signal is that the even and odd components of a non-linear system are uniquely linked to the respective even and odd harmonics in the output DFT spectrum. When applying a full multisine, it is impossible to determine whether a non-linear system provides only even, only odd or both even and odd non-linear components. The only disadvantage in applying an odd multisine compared to applying a full multisine is the loss of a factor two in the frequency resolution.

With an odd multisine as excitation signal, the even non-linear contributions inside and outside the excited frequency range can be determined, as well as the odd non-linear contributions outside the excited frequency range. For determining the odd non-linear contributions within the excited frequency range, some excited odd harmonics can be sacrificed. The measured signal level at these odd detection lines provides the odd non-linear contributions within the excited frequency range. The method according to embodiments of the present invention may be applied with an odd random phase multisine with random harmonic grid $U_{odd,r}$:

$$u_{odd,r}(t) = \sum_{k=1}^{\frac{N}{2}-1} A_{2k-1} \cos\left((2k-1)\omega_0 t + \Phi_{2k-1}\right)$$

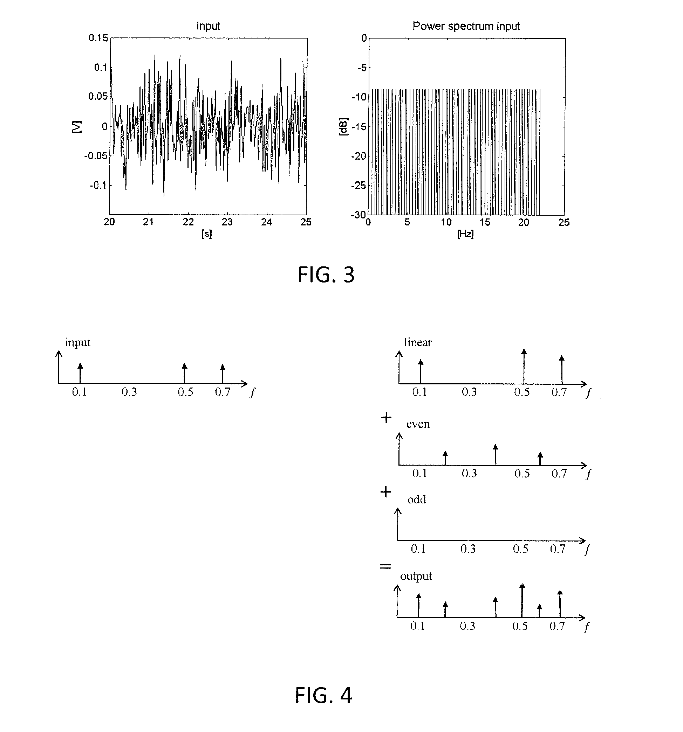

An odd multisine $U_{odd,r}$ is illustrated in the time domain in FIG. 3, left hand side, and in the frequency domain at the right hand side of FIG. 3.

As an example only, experiments have been performed with an excitation signal u(t) with following design parameters:

- frequency interval from 0.5-21.9 Hz; in alternative embodiments the frequency interval could for example range from 0.1-20 Hz;
- frequency resolution $f_0$ of 0.1Hz;
- odd harmonics

$$u_{odd,r}(t) = \sum_{k=1}^{\frac{N}{2}-1} A_{2k-1} \cos\left((2k-1)\omega_0 t + \Phi_{2k-1}\right)$$

- the odd detection lines were chosen with a $F_{group}$ =3; meaning that one odd detection line is randomly selected in every group of three subsequent odd harmonics;
- equal amplitude $A_k$;
- the phase uniformly distributed in the 0-2π interval.

The odd random phase multisine $U_{odd,r}(t)$ as indicated above provides a good linear approximation of the respiratory impedance.

It is to be noted that logarithmic distributions of the detection lines are useful when the frequency band of interest covers several decades. This is useful if the respiratory impedance is analyzed on a broader band (e.g. 0.1-500 Hz), hence also in the frequency range where it is studied according to the prior art. However, in accordance with embodiments of the present invention, non-linearities are studied, where in accordance with the prior art only linear affects are taken into account.

From FIG. 4, one can easily understand the principle of detecting nonlinearities. The left hand side of this figure illustrates an odd excitation signal u(t) exciting at 0.1, 0.5 and 0.7 Hz. The bottom right hand side of FIG. 4 shows the output signal of the non-linear system, where signals are detected at 0.1, 0.2, 0.4, 0.5, 0.6 and 0.7 Hz. This output signal consists of a combination of linear components (i.e. the components which were also present in the input signal, thus for the embodiment illustrated components at 0.1, 0.5 and 0.7 Hz), even components (generated by non-linearities in the system, detected at 0.2, 0.4 and 0.6 Hz), and optionally also odd components other than the ones generated by the excitation signal u(t) (none in the example illustrated).

The acquired information (output signal) can be obtained in one or more experiments. Each time a new experiment is made, another random phase multisine u(t) is sent to the system under observation, hence providing extra information.

The standard procedure for measuring an impulse response function g(t) of a linear system to an excitation signal u(t) is based on the correlation analysis

$$R_{yu}(t) = g(t) * R_{uu}(t)$$

with y(t) the output signal, * the convolution product, and $R_{yu}(t)$ and $R_{uu}(t)$ the cross- and autocorrelation, respectively:

$$R_{yu}(\tau) = E\{y(t)u(t-\tau)\}$$

and

$$R_{uu}(\tau) = E\{u(t)u(t-\tau)\}$$

For random excitations the solution of the above equation minimizes the mean squared error (MSE) between the output y(t) of the linear system and the impulse response to the excitation signal u(t)

$$E\{\|y(t) - g(t) * u(t)\|^2\}$$

with respect to g(t). Taking the Fourier transform gives that

$$G(j\omega) = \frac{S_{YU}(j\omega)}{S_{UU}(j\omega)}$$

where the cross-power spectrum $S_{YU}(j\omega)$, the auto-power spectrum $S_{UU}(j\omega)$ and the frequency response function $G(j\omega)$ are the Fourier transforms of $R_{yu}(t)$, $R_{uu}(t)$ and g(t), respectively.

The best linear approximation (BLA) of a nonlinear system $g_{BLA}(t)$ minimizes the mean squared error (MSE) between the true (shifted) output of the nonlinear system *y(t) - E{y(t)}* and the output of the approximated linear model $g_{BLA}(t)$ * (u(t) - E{u(t)})

$$E\{\|(y(t) - E\{y(t)\}) - g_{BLA}(t) * (u(t) - E\{u(t)\})\|^2\}$$

This best linear approximation is only defined for a pre-determined class of excitation signals. In the frequency domain, the solution to the above minimalisation problem is given by

$$G_{BLA}(j\omega) = \frac{S_{YU}(j\omega)}{S_{UU}(j\omega)}$$

wherein the cross-power spectrum $S_{YU}(j\omega)$, the auto-power spectrum $S_{UU}(j\omega)$ and the frequency response function $G_{BLA}$

(jω) are the Fourier transforms of $R_{yu}(t)$, $R_{uu}(t)$ and $g_{BLA}(t)$, respectively.

For periodic excitation signals u(t) this formula is approximated by,

$$\hat{G}_{BLA}(j\omega_k) = \frac{1}{M} \sum_{m=1}^{M} \frac{Y^{[m]}(k)}{U^{[m]}(k)}$$

where $X^{[m]}(k)$ is the DFT spectrum of the m-th multisine realization x(t), and M are the number of realizations of the random multisine (number of iterative experiments). The random nature of the multisine increases the variability of the estimated BLA. Hence, a large number of averages M is preferred to obtain an accurate estimate. Therefore an iterative method may be applied. As M tends to infinity, the variability of the estimate tends to zero.

It has been shown by J. Schoukens et al., "System identification. A frequency approach.", New Jersey, IEEE press, 2001, that the measured best linear approximation $G_{BL}A\ (j\omega_k)$ (corrupted with nonlinearities and noise) of a wide class of nonlinear systems, obtained using a random phase multisine, can be written as

$$\hat{G}_{BLA}(j\omega_k) = G_{BLA}(j\omega_k) + G_S(j\omega_k) + N_G(j\omega_k)$$

with $G_{BLA}(j\omega_k)$ the best linear approximation (BLA) ("true BLA") of the nonlinear system, $G_S(j\omega_k)$ the zero mean stochastic nonlinear contributions and $N_G(j\omega_k)$ the measurement noise. The stochastic non-linear component $G_s$ is dependent on the power spectrum and the probability density function of the excitation signal, and on the odd and even non-linearities. The stochastic nonlinear contributions $G_S(j\omega_k)$ act as circular complex noise for the noise on G $N_G$ sufficiently large. Hence when applying the random multisine over different measurements, the stochastic nonlinear contributions $G_S(j\omega_k)$ cannot be distinguished from the measurement noise $N_G(j\omega_k)$. The contribution of $G_S$ can be suppressed by averaging the measurements over a large number M of different phase-realisations of a multisine. The contribution of $N_G$ can be suppressed by a longer measurement over multiple periods, as well as by averaging over the M phase realizations. However, longer measurements are not preferred as they tend to be annoying for the subject 10 under test.

In practice, the best linear approximation of a nonlinear system can be written as:

$$G_{BLA}(j\omega_k) = G_0(j\omega_k) + G_B(j\omega_k)$$

where Go is the linear contribution of the system and the $G_B$ is the nonlinear bias-contribution. The systematic nonlinear contribution $G_B$ depends on the power-spectrum and the probability density function of the excitation signal, as well as on the even and odd nonlinearities. This contribution cannot be reduced by longer measurement time, hence can be detected and quantified.

In the time domain, the output y(t) of a system excited by a random phase multisine can be written as

$$y(t) = g_{BLA}(t) * u(t) + y_s(t)$$

wherein $g_{BLA}(t)$ is the impulse response of the best linear approximation (BLA), and $y_s(t)$ is the contribution in the output signal as a result of the stochastic non-linear disturbance.

Now, nonparametric methods are discussed for identifying the best linear approximation (BLA) and the variance of the stochastic nonlinear contributions $G_s(j\omega_k)$ and the disturbing noise $N_G(j\omega_k)$ from noisy input-output observations of a nonlinear system excited by random phase multisines. Two measurement procedures are presented:

1) a robust method (slow) that measures the stochastic nonlinear contributions via different random phase multisine experiments; and

2) a fast method, which estimates the nonlinear contributions in a single experiment, with a specially designed random phase multisine. The information about the stochastic nonlinear distortions is obtained via the detection lines (non-excited harmonics) in the output DFT spectrum. To compensate for spectral impurity of the input, a first order correction may be applied to the output DFT spectrum. The use of an odd random phase multisine with random harmonic grid also allows a classification of the nonlinear distortions in even and odd contributions in the corrected output DFT spectrum.

The advantages of both methods can be combined by using different random phase realizations of an odd multisine with random harmonic grid. The only drawback is an increase in measurement time.

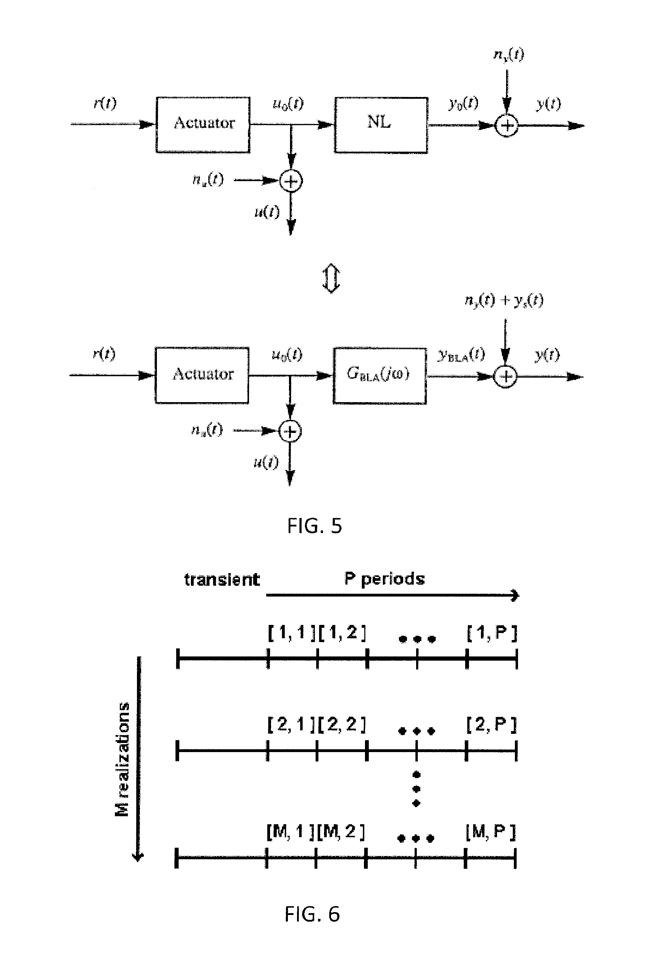

The representation of the best linear approximation is illustrated with FIG. 5. In the top diagram, noisy input/output measurements are illustrated of a nonlinear system driven by an actuator with extended Gaussian input r(t). In the bottom diagram the corresponding best linear approximation is illustrated, where the nonlinear contributions have been separated from the output signal (detected).

The measurement procedure for the robust method is illustrated in FIG. 6: P periods each with length N are measured of the steady state response to an odd random phase multisine. This means, as also illustrated in FIG. 6, that measurements are only performed after a transient period. This experiment is repeated M times for M different odd random phase multisine realizations.

Two cases are possible: 1/ the input $u_0(t)$ is exactly known and the output y(t) is measured with noise; and 2/ the input u(t) and the output y(t) are measured and an exactly known reference signal r(t) exists.

For the first case , having P multisine periods an M realizations, as illustrated in FIG. 6, the algorithm according to embodiments of the present invention can be generalized as follows:

1) chose a frequency resolution $f_0$, the excited harmonics k, the amplitudes $A_k$, and the RMS (root mean square) value of the random multisine r(t) (u(t) = r(t) as input signal is known).
2) make a random choice of the phases of the non-zero harmonics of the random phase multisine such that the variance of the phase is uniformly distributed in the 0-2n interval and obtain r(t).
3) apply the excitation r(t) to the actuator and measure P frequency response functions from P≥2 consecutive periods of the steady state response.
4) repeat steps 2) and 3) for a plurality of times, e.g. M>6 or 7 times.

Since $n_y(t)$ (noise on the output measurement) is a stochastic process and y(t) is a periodic signal, depending on the phase realization of the reference signal r(t), the FRF (frequency response function) of the m-th realization and the p-th period is related to the BLA (best linear approximation) the stochastic distortions and the output noise as in:

$$G^{[m,p]}(j\omega_k) = \frac{Y^{[m,p]}(k)}{U_0^{[m]}(k)} = G_{BLA}(j\omega_k) + \frac{Y_S^{[m]}(k)}{U_0^{[m]}(k)} + \frac{N_Y^{[m,p]}(k)}{U_0^{[m]}(k)}$$

This shows that the sample variance over the P periods only depends on the output noise, while the sample variance over the M realizations depends on both the stochastic nonlinear distortions and the output noise. From the MxP noisy FRFs one can calculate for each experiment (=random phase multisine realization) the average FRF and its sample variance:

$$\hat{G}^{[m]}(j\omega_k) = \frac{1}{P} \sum_{p=1}^{P} G^{[m,p]}(j\omega_k) \qquad \hat{\sigma}^2_{\hat{G}^{[m]}}(k) = \sum_{p=1}^{P} \frac{|G^{[m,p]}(j\omega_k) - \hat{G}^{[m]}(j\omega_k)|^2}{P(P-1)}$$

Additional averaging over the M experiments gives the final FRF for the BLA and its total variance (output and noise), as in:

$$\hat{G}_{BLA}(j\omega_k) = \frac{1}{M} \sum_{m=1}^{M} \hat{G}^{[m]}(j\omega_k) \qquad \hat{\sigma}^2_{\hat{G}_{BLA}}(k) = \sum_{m=1}^{M} \frac{|\hat{G}^{[m]}(j\omega_k) - \hat{G}_{BLA}(j\omega_k)|^2}{M(M-1)}$$

from where an improved estimate of the noise variance can be obtained:

$$\hat{\sigma}^2_{\hat{G}_{BLA,n}}(k) = \frac{1}{M^2} \sum_{m=1}^{M} \hat{\sigma}^2_{\hat{G}^{[m]}}(k)$$

Finally, the variance of the nonlinear distortions is given by

$$var(G_S(j\omega_k)) \approx M(\hat{\sigma}^2_{\hat{G}_{BLA}}(k) - \hat{\sigma}^2_{\hat{G}_{BLA,n}}(k))$$

This variation gives an idea upon the importance of the nonlinear contributions in the observed system.

For the second case, when r(t) is known, the input/output signal spectrum can be projected on this reference signal:

$$U_R^{[m,p]}(k) = \frac{U^{[m,p]}(k)}{R^{[m]}(k)}$$

$$Y_R^{[m,p]}(k) = \frac{Y^{[m,p]}(k)}{R^{[m]}(k)}$$

Calculating sample means and their (co-)variances over the P periods, gives:

$$\hat{U}_R^{[m]}(k) = \frac{1}{P} \sum_{p=1}^{P} U_R^{[m,p]}(k), \hat{Y}_R^{[m]}(k) = \frac{1}{P} \sum_{p=1}^{P} Y_R^{[m,p]}(k)$$

$$\hat{\sigma}^2_{\hat{U}_R^{[m]}}(k) = \sum_{p=1}^{P} \frac{|U_R^{[m,p]}(k) - \hat{U}_R^{[m]}(k)|^2}{P(P-1)}, \hat{\sigma}^2_{\hat{Y}_R^{[m]}}(k) = \sum_{p=1}^{P} \frac{|Y_R^{[m,p]}(k) - \hat{Y}_R^{[m]}(k)|^2}{P(P-1)}$$

$$\hat{U}_R(k) = \frac{1}{M} \sum_{m=1}^{M} \hat{U}_R^{[m]}(k), \hat{Y}_R(k) = \frac{1}{M} \sum_{m=1}^{M} \hat{Y}_R^{[m]}(k)$$

$$\hat{\sigma}^2_{\hat{U}_R}(k) = \sum_{m=1}^{M} \frac{|\hat{U}_R^{[m]}(k) - \hat{U}_R(k)|^2}{M(M-1)}, \hat{\sigma}^2_{\hat{Y}_R}(k) = \sum_{m=1}^{M} \frac{|\hat{Y}_R^{[m]}(k) - \hat{Y}_R(k)|^2}{M(M-1)}$$

$$\hat{\sigma}^2_{\hat{U}_{R,n}}(k) = \frac{1}{M^2} \sum_{m=1}^{M} \hat{\sigma}^2_{\hat{U}_R^{[m]}}(k), \hat{\sigma}^2_{\hat{Y}_{R,n}}(k) = \frac{1}{M^2} \sum_{m=1}^{M} \hat{\sigma}^2_{\hat{Y}_R^{[m]}}(k)$$

with similar notations as before. From here, the estimations for the input/output variation and input/output noise variation are given by:

$$\hat{\sigma}_{\hat{Y}_R^{[m]}\hat{U}_R^{[m]}}(k) = \sum_{p=1}^{P} \frac{(Y_R^{[m,p]}(k) - \hat{Y}_R^{[m]}(k))\overline{(U_R^{[m,p]}(k) - \hat{U}_R^{[m]}(k))}}{P(P-1)}$$

and if an averaging is performed over M realizations, the following is obtained

$$\hat{\sigma}_{\hat{Y}_R\hat{U}_R}(k) = \sum_{m=1}^{M} \frac{(Y_R^{[m]}(k) - \hat{Y}_R(k))\overline{(U_R^{[m]}(k) - \hat{U}_R(k))}}{M(M-1)}$$

and from there it follows that $\quad \hat{\sigma}^2_{\hat{Y}_R\hat{U}_R,n} = \dfrac{1}{M^2}\displaystyle\sum_{m=1}^{M} \hat{\sigma}^2_{\hat{Y}_R^{[m]}\hat{U}_R^{[m]}}(k)$

The BLA is given by

$$\hat{G}_{BLA}(j\omega_k) = \frac{\hat{Y}_R(k)}{\hat{U}_R(k)}$$

with the total variance given by

$$\hat{\sigma}^2_{\hat{G}_{BLA}} = |\hat{G}_{BLA}(j\omega_k)|^2 \left( \frac{\hat{\sigma}^2_{\hat{Y}_R}(k)}{|\hat{Y}_R(k)|^2} + \frac{\hat{\sigma}^2_{\hat{U}_R}(k)}{|\hat{U}_R(k)|^2} - 2Re(\frac{\hat{\sigma}^2_{\hat{Y}_R\hat{U}_R}(k)}{\hat{Y}_R(k)\overline{\hat{U}_R(k)}}) \right)$$

and the total noise variance given by

$$\hat{\sigma}^2_{\hat{G}_{BLA,n}} = |\hat{G}_{BLA}(j\omega_k)|^2 \left( \frac{\hat{\sigma}^2_{\hat{Y}_R,n}(k)}{|\hat{Y}_R(k)|^2} + \frac{\hat{\sigma}^2_{\hat{U}_R,n}(k)}{|\hat{U}_R(k)|^2} - 2Re(\frac{\hat{\sigma}^2_{\hat{Y}_R\hat{U}_R,n}(k)}{\hat{Y}_R(k)\overline{\hat{U}_R(k)}}) \right)$$

Hence, the variance of the stochastic nonlinear distortions can be estimated as:

$$var(G_S(j\omega_k)) \approx M(\hat{\sigma}^2_{\hat{G}_{BLA}}(k) - \hat{\sigma}^2_{\hat{G}_{BLA,n}}(k))$$

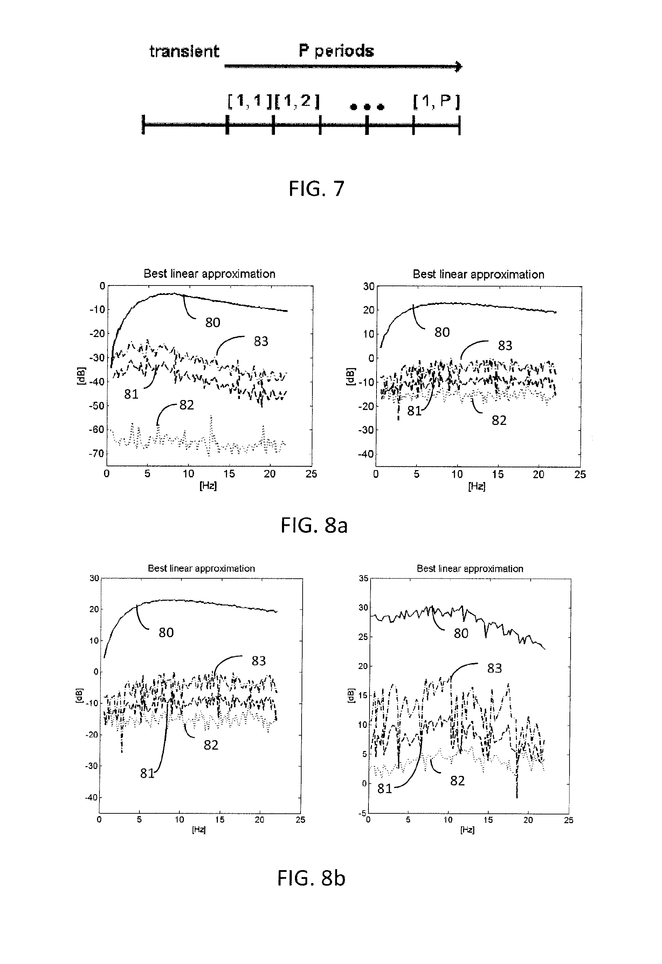

An advantage of the robust method is that there is no limitation in frequency resolution. Another advantage of the robust method is that there is no limitation on the input signal-to-noise ratio. Yet another advantage of the robust method is that any type of random phase multisine can be applied. Still another advantage of the robust method is that there are only small uncertainties on the estimates, in view of the average which is taken over the measurements.
For the fast method, a single measurement is performed (M=1), as illustrated in FIG. 7.

The measurement algorithm remains the same as for the robust method. For each period P the input/output spectra with noise can be estimated. From these, the averaged input/output spectra can be obtained, and their corresponding (co-)variances:

$$\hat{U}(k) = \frac{1}{P}\sum_{p=1}^{P} U^{[p]}(k), \hat{Y}(k) = \frac{1}{P}\sum_{p=1}^{P} Y^{[p]}(k)$$

$$\hat{\sigma}^2_{\hat{U},n}(k) = \sum_{p=1}^{P} \frac{|\hat{U}^{[p]}(k) - \hat{U}(k)|^2}{P(P-1)}, \hat{\sigma}^2_{\hat{Y},n}(k) = \sum_{p=1}^{P} \frac{|\hat{Y}^{[p]}(k) - \hat{Y}(k)|^2}{P(P-1)}$$

$$\hat{\sigma}^2_{\hat{Y}\hat{U},n}(k) = \sum_{p=1}^{P} \frac{(\hat{Y}^{[p]}(k) - \hat{Y}(k))\overline{(\hat{U}^{[p]}(k) - \hat{U}(k))}}{P(P-1)}$$

The best linear approximation (BLA) and noise variance can be estimated as follows:

$$\hat{G}_{BLA}(j\omega_k) = \frac{\hat{Y}(k)}{\hat{U}(k)}$$

$$\hat{\sigma}^2_{\hat{G}_{BLA},n} = |\hat{G}_{BLA}(j\omega_k)|^2 \left( \frac{\hat{\sigma}^2_{\hat{Y},n}(k)}{|\hat{Y}(k)|^2} + \frac{\hat{\sigma}^2_{\hat{U},n}(k)}{|\hat{U}(k)|^2} - 2Re\left(\frac{\hat{\sigma}^2_{\hat{Y}\hat{U},n}(k)}{\hat{Y}(k)\overline{\hat{U}(k)}}\right) \right)$$

A null hypothesis test is a hypothesis that might be falsified by using a test of observed data. Such test comprises formulating a null hypothesis, collecting measurement data, and calculating a measure of how probable that measurement data was, assuming the null hypothesis were true. If the data appears very improbable (usually defined as a type of data that should be observed less than 5% of the time) then the experimenter concludes that the null hypothesis is false. If the data looks reasonable under the null hypothesis, then no conclusion is made. In this case, the null hypothesis could be true, or it could still be false; the data gives insufficient evidence to make any conclusion. Via such null hypothesis test, one can check if the detection lines (non-excited harmonics) in the input/output spectra do or do not contain signal energy:

$$|\hat{X}(k)|^2/\hat{\sigma}^2_{\hat{X},n}(k) \leq F_{0.95,2,2P-2}$$

With $F_{0.95,2,2P-2}$ the 95% of a $F_{0.95,2,2P-2}$ distributed variable (Matlab Statistics Toolbox) is the null hypothesis $Xo(k) = 0$ accepted; otherwise rejected. If the input detection lines contain no energy, (null hypothesis accepted) then the presence of the signal energy at the output detection lines is directly linked to the nonlinear behavior of the system. If the input detection lines do contain signal energy (null hypothesis rejected) then the signal energy at the output detection lines is at least partially due to input energy at those frequencies, and a direct link with the nonlinear behavior of the system cannot be established. However, if the spectral impurity at the input is small, a correction of its influence on the output can be done as follows:

1) the BLA at the non-excited in-band harmonic $\omega_l$ is calculated via linear interpolation of the BLA at the closest

excited harmonics $\omega_k < \omega_l$ and $\omega_m > \omega_l$:

$$\hat{G}_{BLA}(j\omega_l) = \frac{(m-l)\hat{G}_{BLA}(j\omega_k) + (l-k)\hat{G}_{BLA}(j\omega_m)}{m-k}$$

2) the output spectrum at the non-excited harmonic $\omega_l$ is corrected as

$$\hat{Y}_c(l) = \hat{Y}(l) - \hat{G}_{BLA}(j\omega_l)\hat{U}(l)$$

This last relation can be motivated as follows: if the input signal amplitude at the non-excited frequencies (detection lines) is small compared with that at the excited frequencies (everything measured at the non-excited frequencies will not be the effect of the input, but rather will be the effect of non-linearities in the system), then the nonlinear contributions of input detection lines at the output spectrum can be neglected with respect to those at the excited input harmonics Hence, the linear contribution of the input detection lines at the output is dominant and non-linear effects can be removed via this last relation.

Finally, the level of the nonlinear distortion on the BLA measurement is compensated for the presence of some disturbing power at input lines (that should be zero) by estimating their linear contribution to the output. Next this is subtracted from the output such that at the output only the nonlinear contribution remains (first order correction).

1) The level of the stochastic nonlinear distortions may be extrapolated to the excited harmonics via linear interpolation of the power of the closest in-band detection lines l<k and m>k:

$$|\hat{Y}_c(k)|^2 = \frac{(m-k)|\hat{Y}_c(l)|^2 + (k-l)|\hat{Y}_c(m)|^2}{m-l}$$

With an odd multisine only odd detection lines are used.

2) Since $\hat{Y}_o(l)$ contains the contribution of the input and output noise, the ratio

$$\hat{\sigma}^2_{\hat{G}_{BLA}}(k) = \frac{|\hat{Y}_c(k)|^2}{|\hat{U}(k)|^2}$$

where k denotes the excited harmonic, can be used as an estimate of the total variance (stochastic nonlinear distortion + input/output noise) of BLA;

3) Finally, the variance of the stochastic nonlinear distortions can be calculated as:

$$var(G_S(j\omega_k)) \approx (\hat{\sigma}^2_{\hat{G}_{BLA}}(k) - \hat{\sigma}^2_{\hat{G}_{BLA,n}}(k))$$

It is to be noted that, contrary to the robust method, in this fast method all variances are referred with respect to one multisine realization (M=1).
FIG. 18 represents, as an example, the BLA of a calibration pipe. The left hand part of FIG. 18 shows the results for a fast method (1 realisation), the right hand part shows the results for a robust method (4 measurements). It can be seen that there is no interference/noise at low frequencies (there is no corrupting noise from breathing since only a pipe is measured, without patient).

An advantage of the fast method is that it takes only a small measurement time, which is appreciated by the subject under test. Another advantage of the fast method is that a classification of the nonlinear distortion in even/odd contributions within and out of the excitation band is possible. Yet another advantage of the fast method is that it is easy to apply validation of the input (im-)purities.

By applying any of the above measurement methods on an FOT-device with input u(t), the applied voltage to the loudspeaker 11, and output q(t), the resulting flow, one can detect the nonlinear distortion in the measurement instrumentation. FIG. 8a illustrates the measurement of the BLA of the device impedance $Z_{device}$ of the prior art device I2M (left) and of an IRs8 device (loudspeaker-driven) according to embodiments of the present invention on a calibration tube. FIG. 8b illustrates the measurement of the BLA of the device impedance $Z_{device}$ of the loudspeaker-driven device according to a first embodiment of the present invention (left) and of a pump-driven device according to a second embodiment of the present invention on a calibration tube. In both FIG. 8a and FIG.8b, graph 80 illustrates the best linear approximation, graph 81 shows the total variance (sum noise and stochastic non-linear distortion), graph 82 shows the noise variance and graph 83 shows the variance of the stochastic non-linear distortion with respect to one multisine realization. FIG. 8 shows the relatively big influence of noise (graph 82) in an IRs8 device according to embodiments of the present invention, which is reflected in the slightly noisier result for the respiratory impedance in FIG. 2. The nonlinear distortion (graph 83) in the prior art device I2M is smaller for frequencies > 15 Hz, but larger for frequencies < 6 Hz, compared to the loudspeaker-driven device according to embodiments of the present invention (FIG. 8a). It can be appreciated from FIG. 8b, comparing a loudspeaker-driven device with a piston-driven device according to embodiments of the present invention, that fake non-linear effects' at frequencies below 6 Hz are coming from the inability of the loudspeaker to adequately send such low frequency oscillations (see also FIG. 15). Hence it can be observed that a more realistic effect is obtained at lower frequencies with a piston-driven device, the amplitude of the signal being more or less constant throughout the frequency interval. This is the main reason for the better results of the piston-driven device according to embodiments of the present invention at lower frequencies. Another improvement in a device according to embodiments of the present invention is that the power of the excited input is higher, hence a better signal-to-noise ratio is achieved (especially for low frequencies, where the breathing is an important source of noise).

The applied excitation signal can be optimized by making a nonlinear variance analysis.

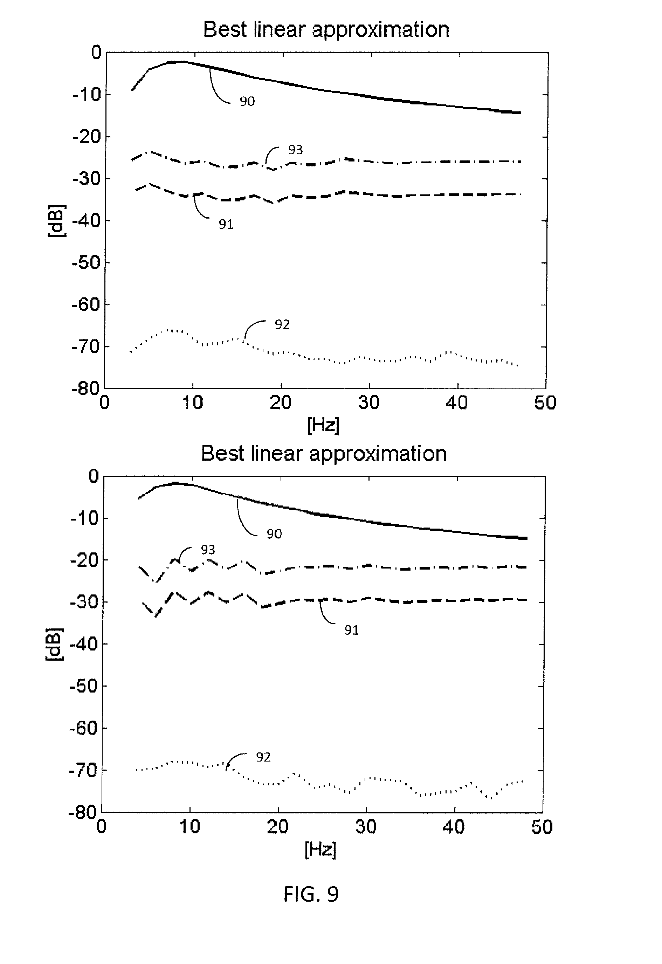

FIG. 9 illustrates measurements of the best linear approximation of the device impedance $Z_{device}$ of a prior art device I2M with odd multisine $U_{odd}$ (top) and even multisine $U_{even}$ (bottom). Graphs 90 illustrate the BLA; graphs 91 illustrate the total variance (sum noise and stochastic nonlinear distortion); graphs 92 illustrate the noise variance; and graphs 93 illustrate the variance of the stochastic nonlinear distortion with respect to one multisine realization. FIG. 9 shows that with an even multisine, it is possible to distinguish between the even and odd nonlinearities (graph 93). The even nonlinearities are higher than the nonlinear contribution in the output of the odd multisine. This is mostly visible at higher frequencies, where a cumulative effect of distortions is coming from the lower excitation frequencies. By choosing an odd multisine as excitation signal, the variance of the stochastic nonlinear distortion, and consequently the total variance (graph 91), is reduced with a factor three. The noise variation (graph 92) is unaffected by the excitation signal (as expected).

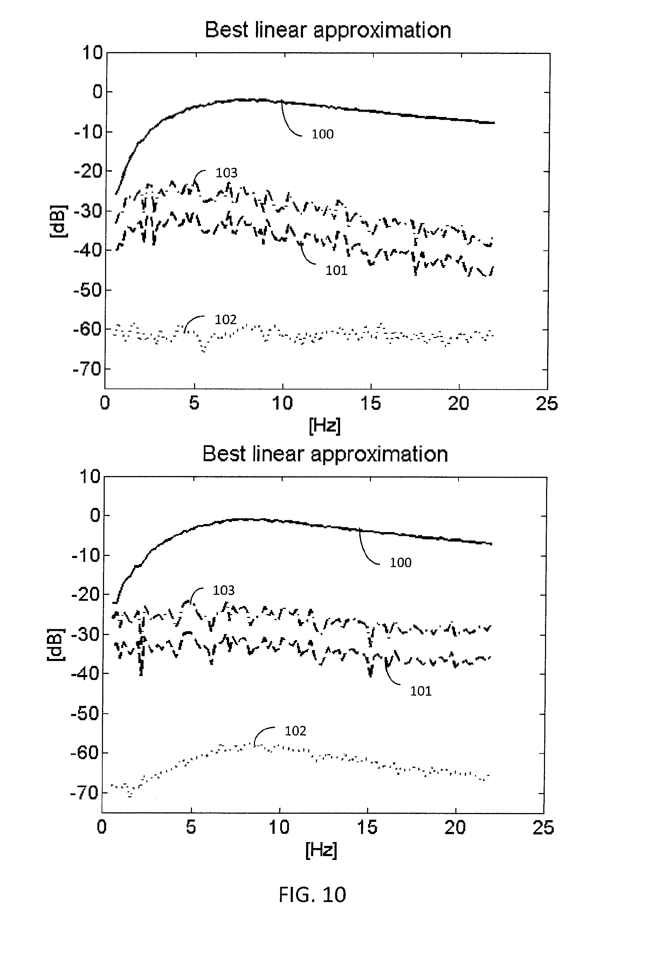

A better signal-to-noise ratio can be obtained by enhancing the excitation signal. However, this generally has an effect on the nonlinear distortions in the system, e.g. through saturation of the loudspeaker 11 or the amplifier. When the enhancement of the signal is too big, the total variance of the BLA will increase through an increased variance of the stochastic nonlinear distortion. This can be observed in FIG. 10. FIG. 10 illustrates measurements of the BLA of the device impedance $Z_{device}$ of I2M with ½ $U_{odd}$ (top) and 2$U_{odd}$ (bottom). Graphs 100 illustrate the BLA; graphs 101 illustrate the total variance (sum of noise and stochastic nonlinear distortion); graphs 102 illustrate the noise variance; and graphs 103 illustrate the variance of the stochastic nonlinear distortion with respect to one multisine realization.

Sometimes an enhancement of the lower frequencies is applied in the multisine identification signal. Consequently, this increases the nonlinear distortion at these lower frequencies and a correct interpretation of the corresponding results can be compromised.

A comparison of the nonlinear effects in the respiratory impedance obtained with an IRs8 device according to embodiments of the present invention and a prior art I2M device reveal that the results are influenced by the nonlinear effects in the measurement instrumentation. This implies that the results for the nonlinear effects in the respiratory impedance of different patients can only be compared with measurements made on a same FOT-device.

By applying the measurement methods according to embodiments of the present invention on the respiratory impedance with input q(t), the flow at the mouth, and output p(t), the corresponding pressure, one can detect the nonlinear distortion in the respiratory impedance.

The measurements according to some embodiments of the present invention are disturbed by the nonstationary breathing signal, especially at the lower frequencies. This disturbance may make it difficult to interpret the results at lower frequencies. The most effective method to reduce this effect is to increase the measurement time. Unfortunately, this is not always practically feasable, e.g. a child with asthma has problems to undergo measurements longer than 30 seconds without significant fatigue caused by the resistance of the FOT-device.

In accordance with embodiments of the present invention, a method as recited above may be adapted for minimizing the influence of the breathing signal, which is considered to be noise. For obtaining this, a simple solution has been applied: before any test with a multisine excitation signal u(t), during a few seconds (e.g. 3 to 4 breathing periods, fairly similar to a wavelength of a sinusoidal signal) measurement with no input has been done. This provides a measurement of the breathing of the patient, usually at low frequencies, around 0.15-0.25Hz. The energy of this signal in its spectral power density function will give information upon the breathing frequency. In the design of the excitation signal, e.g. odd multisine, harmonics of the breathing frequency can be avoided. In this way, the contribution of the breathing signal can be separated solely as an extra term in a system according to embodiments of the present invention.

Embodiments of the present invention may be used for assessing the dynamic mechanical status of the lungs of a patient.

The nonlinear analysis according to embodiments of the present invention has been performed on test groups.

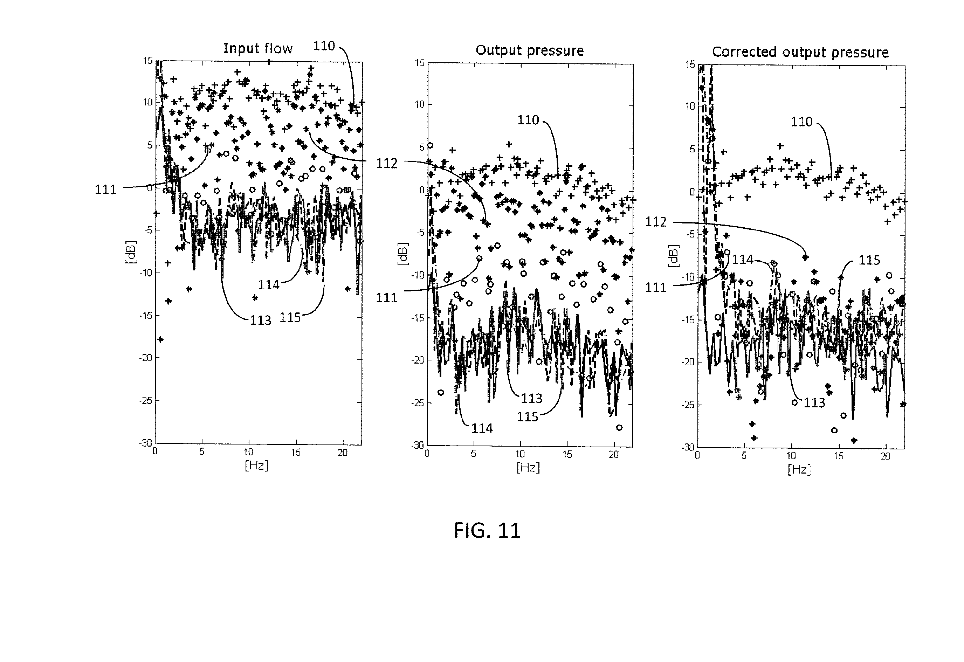

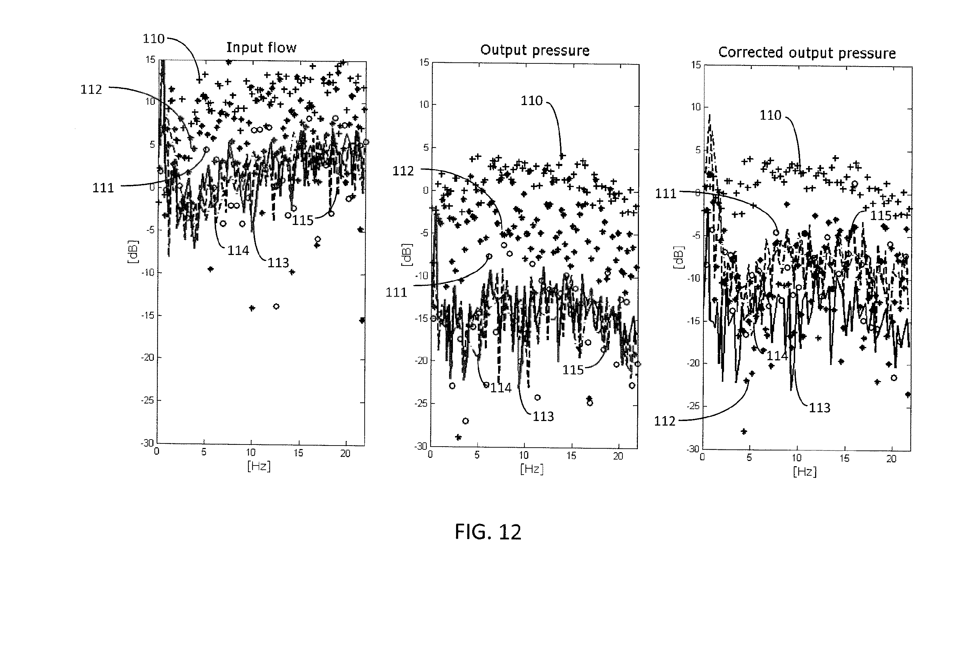

In the first group, the nonlinear effects in the respiratory impedance of children aged 7 to 17 years are compared. FIG. 11 and FIG. 12 illustrate the input flow q(t) (left column), the output pressure p(t) (middle column) and the corrected DFT spectrum (right column) of a 9-year old and a 7-year old healthy patient, respectively. In the graphs, 110 indicates excited odd harmonics, 111 indicates non-excited odd harmonics, 112 indicates non-excited even harmonics, 113 indicates the variance of the excited odd harmonics, 114 indicates the variance of the non-excited odd harmonics and 115 indicates the variance of the non-excited even harmonics. It can be appreciated from FIG. 11 and FIG. 12 that there are differences in the data; however, these differences are not so significant as between healthy-diseased persons (not illustrated). On the other hand, if adults are compared to children, significant differences are found.

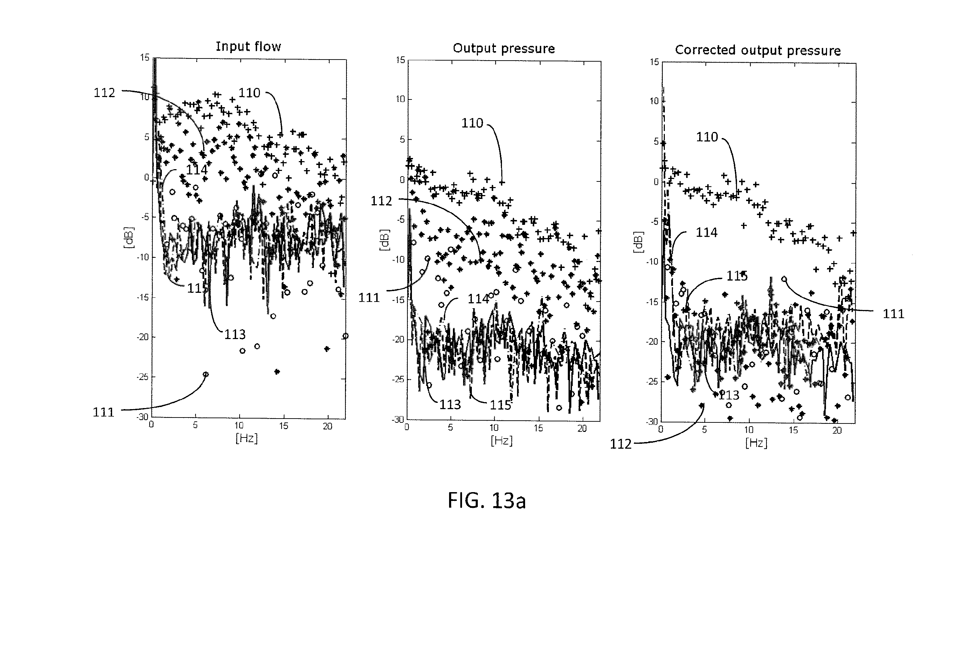

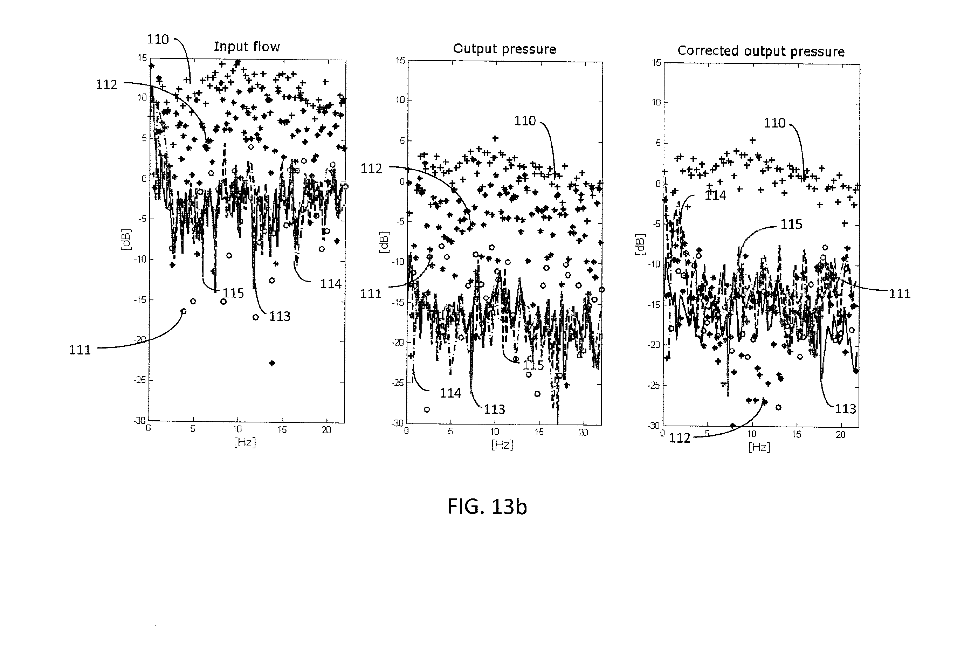

In another group, the nonlinear effects in the respiratory impedance of smokers and nonsmokers aged 23 to 25 years are compared. The smokers were instructed to smoke a cigarette before the measurements. FIG. 13a illustrates the input flow q(t) (left column), the output pressure p(t) (middle column) and the corrected DFT spectrum (right column) of a non-smoker, and FIG. 13b of a smoker. The even and odd nonlinear contributions had a higher level in the corrected output DFT spectrum of a smoker (see FIG. 13b). It can be appreciated that there is a higher resistance in the lungs of a smoker (tissue is more fibrotic, hence less elastic than in a healthy person).

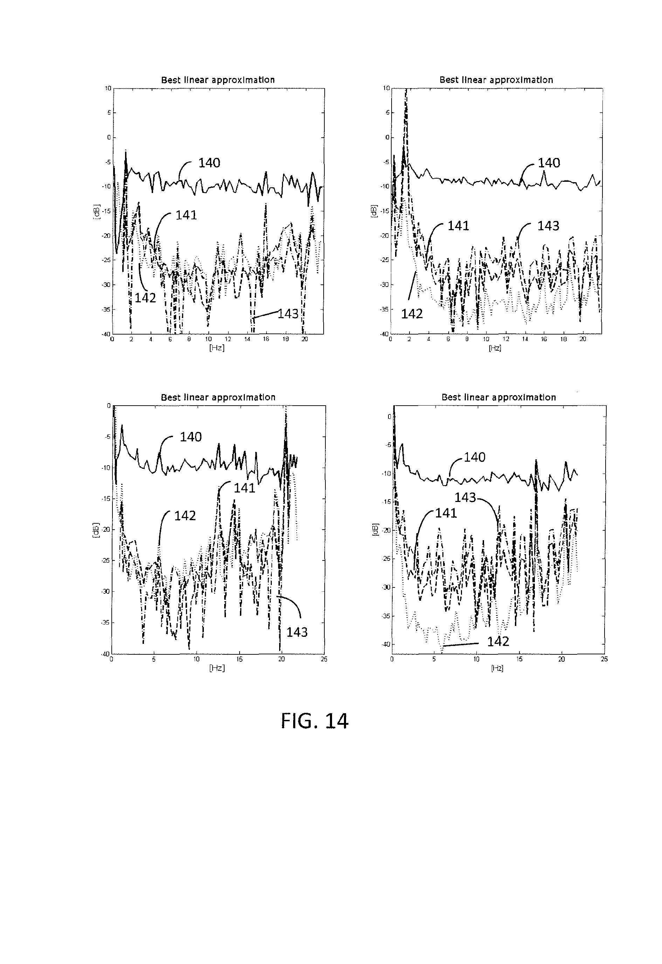

FIG. 14 illustrates the measurement of the BLA of the respiratory impedance $Z_{respiratory}$ of a smoker (top) and a non-smoker (bottom). Graphs 140 illustrate the BLA; graphs 141 illustrate total variance (sum noise and stochastic nonlinear distortion); graphs 142 illustrate noise variance; and graphs 143 illustrate variance of the stochastic nonlinear distortion with respect to one multisine realization. FIG. 14 shows that the variance of stochastic nonlinear distortions (graph 143) is larger in the respiratory impedance of smokers. The difference between smokers and non-smokers increases with decreasing frequency, which implies that valuable information can still be obtained at lower frequencies by reducing the influence of the breathing signal.

While the invention has been illustrated and described in detail in the drawings and foregoing description, such illustration and description are to be considered illustrative or exemplary and not restrictive. The invention is not limited to the disclosed embodiments. It should be noted that the use of particular terminology when describing certain features or aspects of the invention should not be taken to imply that the terminology is being re-defined herein to be restricted to include any specific characteristics of the features or aspects of the invention with which that terminology is associated.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. Method for determining non-linear effects in respiratory impedance of a subject having a respiratory system, the method comprising
   constructing an excitation signal comprising a plurality of excitation frequencies,
   applying the constructed excitation signal to an actuator for applying pressure oscillations to the respiratory system of the subject,
   measuring the frequency response function of the respiratory impedance, and performing a frequency analysis,

determining presence of non-excited harmonics in the frequency response function.

2. Method according to claim 1, furthermore comprising determining a breathing frequency of the subject.

3. Method according to claim 2, wherein determining a breathing frequency of the subject comprises, before any measurement with an excitation signal, measuring the frequency response function of the respiratory impedance with no externally applied input signal.

4. Method according to claim 3, wherein measuring the frequency response function of the respiratory impedance with no externally applied input signal is performed during a few breathing periods.

5. Method according to any of claims 2 to 4, wherein constructing an excitation signal comprises constructing a signal comprising frequencies different from the breathing frequency and harmonics thereof.

6. Method according to any of the previous claims, wherein constructing an excitation signal comprises constructing a random phase odd multisine with random harmonic grid defined by

$$U_{odd,r}(t) = \sum A_{2k-1} cos((2k-1)\omega_0 t + \phi_{2k-1})$$

where in-band harmonics are randomly omitted.

7. Method according to claim 6, wherein constructing an excitation signal comprises

a. selecting a frequency resolution $f_0$, excited harmonics k, amplitudes $A_k$, and the root mean square value of the excitation signal,
b. making a random choice of the phases of the non-zero harmonics of the excitation signal such that the variance of the phase is uniformly distributed in the 0-2n interval and obtaining the excitation signal.

8. Method according to any of the previous claims, wherein constructing an excitation signal comprises constructing a signal in a frequency range between 0.1 Hz and 50.0 Hz.

9. Method according to any of the previous claims, comprising repeating the steps of constructing an excitation signal, applying the constructed excitation signal, measuring the frequency response function of the respiratory impedance and determining presence of non-excited harmonics in the frequency response function.

10. Method according to any of the previous claims, wherein measuring the frequency response function of the respiratory impedance comprises measuring the frequency response function from a plurality of consecutive periods of a steady state response.

11. A controller for determining non-linear effects in respiratory impedance of a subject having a respiratory system, the controller being programmed for constructing an excitation signal comprising a plurality of excitation frequencies, for applying the constructed excitation signal to an actuator for applying pressure oscillations to the respiratory system of the subject, for measuring the frequency response function of the respiratory impedance and performing a frequency analysis, and for determining presence of non-excited harmonics in the frequency response function.

12. An FOT device comprising a controller according to claim 11.

13. An FOT device according to claim 12, wherein the FOT device comprises a piston based pump for generating a pressure oscillation for being applied to a subject.

14. A computer program product for executing any of the methods claimed in any of claims 1 to 10 when executed on a controller associated with an FOT device.

15. A machine readable data storage storing the computer program product of claim 14.

FIG. 1

FIG. 2

FIG. 3

FIG. 4

FIG. 5

FIG. 6

EP 2 384 697 A1

FIG. 7

FIG. 8a

FIG. 8b

Best linear approximation

Best linear approximation

FIG. 9

## Best linear approximation

## Best linear approximation

FIG. 10

FIG. 11

FIG. 12

FIG. 13a

FIG. 13b

FIG. 14

FIG. 15

FIG. 16

FIG. 17

FIG. 18

Europäisches
Patentamt

European
Patent Office

Office européen
des brevets

## EUROPEAN SEARCH REPORT

Application Number

EP 10 16 1953

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | C IONESCU AND R DE KEYSER: "Detection of novel respiratory mechanic parameters by means of forced oscillations", PREPRINTS OF THE 16TH IFAC WORLD CONGRESS, 02524, 2005, pages 1-6, XP002604715, | 1,2,5, 8-12,14, 15 | INV. A61B5/08 A61B5/085 G06F19/00 |
| Y | * section "2.Apparatus and lung function test"figure 1 * * subsections 3.1 and 3.2 of section "3. Signal Processing"figures 2,5 * * section "5. Discussion"; last paragraph * * section "6. Conclusion"; last paragraph * ----- | 3,4 | |
| Y | C IONESCU, K DASAGER, R DE KEYSER: "Estimating respiratory mechanics with constant-phase models in healthy lungs from forced oscillations measurements", STUDIA UNIVERSITATIS "VASILE GOLDIS", SERIA STIINTELE VIETII, vol. 19, no. 1, March 2009 (2009-03), pages 123-131, XP002604716, | 3,4 | |
| A | * sections "measurements" and "excitation signals"; page 124 - page 126 * ----- | 1,11,14, 15 | TECHNICAL FIELDS SEARCHED (IPC) A61B G06F |
| A | US 6 435 182 B1 (LUTCHEN KENNETH R [US] ET AL) 20 August 2002 (2002-08-20) * abstract; claims 1,3,4,5,15 * * column 5, line 35 - column 6, line 52 * ----- | 1,11,14, 15 | |
| A | WO 2005/104944 A1 (UNIV DALHOUSIE [CA]; MAKSYM GEOFFREY N [CA]; LALL CAROLYN A [CA]) 10 November 2005 (2005-11-10) * abstract * * page 8, lines 4-17 * ----- | 1,11,14, 15 | |
| | -/-- | | |

~~The present search report has been drawn up for all claims~~

| Place of search | Date of completion of the search | Examiner |
|---|---|---|
| Munich | 11 March 2011 | Medeiros Gaspar, Ana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................
& : member of the same patent family, corresponding document

EPO FORM 1503 03.82 (P04C01)

**Europäisches Patentamt**
**European Patent Office**
**Office européen des brevets**

## EUROPEAN SEARCH REPORT

**Application Number**

EP 10 16 1953

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2009/113500 A1 (CHEST M I INC [JP]; UNIV TOHOKU [JP]; KUROSAWA HAJIME [JP]; SHIMIZU YO) 17 September 2009 (2009-09-17) * abstract; claims 6,11,12; figures 1,10 * * paragraphs [0010] - [0014], [0018], [0028] - [0033], [0053] - [0055], [0059] * | 1,2, 6-12,14, 15 | |
| E | & EP 2 253 268 A1 (CHEST M I INC [JP]; TOHOKU TECHNO ARCH CO LTD [JP]) 24 November 2010 (2010-11-24) * abstract; claims 6,11,12; figures 1,10 * * paragraphs [0010] - [0014], [0018], [0028] - [0033], [0053] - [0055], [0059] * | 1,2, 6-12,14, 15 | |
| X | ----- WO 03/103493 A1 (MILANO POLITECNICO [IT]; DELLACA RAFFAELE [IT]; ALIVERTI ANDREA [IT];) 18 December 2003 (2003-12-18) * abstract; figure 1 * * page 7, line 28 - page 10, line 27 * * page 11, line 23 - page 12, line 8 * ----- | 1,6,7 | |

TECHNICAL FIELDS SEARCHED (IPC)

~~The present search report has been drawn up for all claims~~

| Place of search | Date of completion of the search | Examiner |
|---|---|---|
| Munich | 11 March 2011 | Medeiros Gaspar, Ana |

Europäisches
Patentamt

European
Patent Office

Office européen
des brevets

**Application Number**

EP 10 16 1953

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☒ Only part of the further search fees have been paid within the fixed time limit. The present European search report has been drawn up for those parts of the European patent application which relate to the inventions in respect of which search fees have been paid, namely claims:

1-10, 12, 14, 15(completely); 11(partially)

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

The Search Division considers that the present European patent application does not comply with the
requirements of unity of invention and relates to several inventions or groups of inventions, namely:

1. claims: 2-5(completely); 1, 8-12, 14, 15(partially)

    Methods for determining non-linear effects in respiractory
    impedance of a subject comprising determining, before any
    measurement with an excitation signal,  the breathing
    subject of the subject by measuring the frequency response
    of the respiratory impedance with no externally applied
    signal
                    ---

2. claims: 6, 7(completely); 1, 8-12, 14, 15(partially)

    Methods for determining non-linear effects in respiractory
    impadance of a subject, comprising constructing an
    excitation signal comprising a random phase multisine wave
    with random harmonic grid wherein band harmonics are
    randomly omitted
                    ---

3. claims: 13(completely); 11, 12(partially)

    FOT device comprising a piston based pump
                    ---

**ANNEX TO THE EUROPEAN SEARCH REPORT
ON EUROPEAN PATENT APPLICATION NO.**

EP 10 16 1953

This annex lists the patent family members relating to the patent documents cited in the above-mentioned European search report.
The members are as contained in the European Patent Office EDP file on
The European Patent Office is in no way liable for these particulars which are merely given for the purpose of information.

11-03-2011

| Patent document cited in search report | | Publication date | Patent family member(s) | | Publication date |
|---|---|---|---|---|---|
| US 6435182 | B1 | 20-08-2002 | NONE | | |
| WO 2005104944 | A1 | 10-11-2005 | AU | 2005237191 A1 | 10-11-2005 |
| | | | CA | 2565625 A1 | 10-11-2005 |
| | | | CN | 1972631 A | 30-05-2007 |
| | | | EP | 1742572 A1 | 17-01-2007 |
| | | | JP | 2007536026 T | 13-12-2007 |
| | | | NZ | 551074 A | 27-08-2010 |
| WO 2009113500 | A1 | 17-09-2009 | CA | 2717318 A1 | 17-09-2009 |
| | | | CN | 101959455 A | 26-01-2011 |
| | | | EP | 2253268 A1 | 24-11-2010 |
| | | | JP | 2009240752 A | 22-10-2009 |
| | | | KR | 20100120204 A | 12-11-2010 |
| | | | US | 2011060237 A1 | 10-03-2011 |
| WO 03103493 | A1 | 18-12-2003 | AU | 2003250829 A1 | 22-12-2003 |
| | | | EP | 1551293 A1 | 13-07-2005 |
| | | | EP | 2044883 A2 | 08-04-2009 |
| | | | IT | MI20021273 A1 | 11-12-2003 |
| | | | US | 2005178385 A1 | 18-08-2005 |
| | | | US | 2008114261 A1 | 15-05-2008 |

For more details about this annex : see Official Journal of the European Patent Office, No. 12/82